# EUROPEAN PATENT APPLICATION

(11) **EP 2 050 756 A1**
(43) Date of publication of application: **22.04.2009**
(21) Application number: 07768326.6
(22) Date of filing: 06.07.2007
(51) Int. Cl.: C07F 9/6574, B32B 15/08, B32B 15/092, C08K 5/5313, C08L 63/00, C09K 21/12, H05K 1/03

(54) **PHOSPHORUS-CONTAINING BENZOXAZINE COMPOUND, PROCESS FOR PRODUCTION THEREOF, CURABLE RESIN COMPOSITION, CURED ARTICLE, AND LAMINATE PLATE**

(30) Priority: 20.07.2006 JP 2006198175
(71) Applicant: SHOWA HIGHPOLYMER CO., LTD., Tokyo 105-0012 (JP)
(72) Inventor: TAKAHASHI, Kentarou, Isesaki-shi, Gunma 372-0833 (JP); LI, Hui, Isesaki-shi, Gunma 372-0833 (JP); KAMATA, Hirotoshi, Isesaki-shi, Gunma 372-0833 (JP)
(74) Representative: Gille Hrabal Struck Neidlein Prop Roos
(86) International application number: PCT/JP2007/063601
(87) International publication number: WO 2008/010430

(57) **Abstract**

The present invention is directed to a compound serving as both a flame retardant and a curing agent (a crosslinking agent) for curable resin, a method for producing the compound, a flame-retardant curable resin composition containing the compound, and a cured product and a laminate sheet having flame retardancy produced through curing the cured resin composition. The compound has a benzoxazine structure and a phosphine oxide structure in a molecule thereof.

## Description

### Technical Field

The present invention relates to a phosphorus-containing benzoxazine compound which is excellent in heat resistance and water resistance and which is a useful curing agent and flame retardant for epoxy resin and phenolic resin; to a method for producing the compound; to a curable resin composition having flame retardancy and containing the compound; and to a thermally cured product and a laminate sheet having flame retardancy. The compound of the invention is suitable for producing a semiconductor encapsulant, a laminate sheet, a coating material, a composite material, etc.

### Background Art

A variety of epoxy resins and phenolic resins are employed as electric and electronic materials. Parts made of these materials are required to have high flame retardancy, which has been imparted thereto by use of a halogen compound. However, use of halogen compounds has become problematic, with a recent trend for reducing impacts on the environment.
As an alternative technique for imparting flame retardancy, there has been employed phosphorus compounds such as phosphate esters (e.g., triphenyl phosphate) and condensed phosphate esters [e.g., 1,3-phenylene bis(di-2,6-xylenylphosphate)]. However, when such a phosphorus compound is added as an addition type flame retardant, the obtained cured product exhibits a drop in heat resistance, particularly in Tg.
In order to solve the problem, there has been proposed techniques employing a reactive phosphorus compound which is produced by reacting an epoxy resin having a novolak epoxy resin content of 20% by mass or higher, a quinone compound, and a phosphorus compound (e.g., 9,10-dihydro-9-oxa-10-phosphaphenanthrene-10-oxide or diphenylphosphine oxide), whereby physical properties such as flame retardancy and water resistance are improved (for example, Patent Documents 1 to 3). These techniques are based on improvement of heat resistance, flame retardancy, etc. of molded articles through employment of an epoxy resin modified by a phosphorus compound.

Meanwhile, there has also been proposed use of a compound having a benzoxazine structure as a curing agent for a curable resin composition (for example, Patent Documents 4 to 7). Also, some proposed techniques employ a phosphorus compound having a benzoxazine structure (for example, Patent Document 8). However, the amine compound forming the benzoxazine structure is a monoamine compound, which has no reactive group generally employed in curing reaction and which does not ensure satisfactory heat resistance of the cured products.
Patent Document 1: JP 4-11662 A
Patent Document 2: JP 11-279258 A
Patent Document 3: JP 2000-309623 A
Patent Document 4: JP 2001-220455 A
Patent Document 5: JP 2001-329049 A
Patent Document 6: JP 2003-147165 A
Patent Document 7: JP 2004-352670 A
Patent Document 8: JP 2004-528285 A

### Disclosure of the Invention

### Problems to be Solved by the Invention

The epoxy resins modified by a phosphorus compound disclosed in Patent Documents 1 to 3 have drawbacks. For example, the phosphorus content thereof is as low as 2 to 4% by mass, and a large amount of such epoxy resin is required for providing a phosphorus-containing flame-resistant resin composition. Therefore, physical properties of the resultant cured resin product depend on the physical properties of the epoxy resin modified by a phosphorus compound. Thus, properties such as glass transition temperature and adhesion are unsatisfactory, which is problematic.
The compounds having a benzoxazine structure and disclosed in Patent Documents 4 to 7 have curing or crosslinking ability, but have no flame retardancy. Therefore, a flame retardant is required to be added.
The compound having a phosphorus-containing benzoxazine structure and disclosed in Patent Document 8 has curing or crosslinking ability and flame retardancy. However, the amine compound forming the benzoxazine structure is a monoamine compound, which has no reactive group generally employed in curing reaction and which does not ensure satisfactory heat resistance of the cured products.
In view of the foregoing, objects of the present invention for solving the problems involved in conventional techniques are to provide a novel compound serving as both a curing agent for curable resin and a flame retardant, a method for producing the compound, a curable resin composition containing the compound, and a cured product and a laminate sheet having flame retardancy produced through curing the cured resin composition.

### Means for Solving the Problems

The present inventors have carried out extensive studies in order to attain the aforementioned objects, and have found that the objects can be attained through employment of a reactive phosphorus compound. It has in a molecule thereof a phosphorus-containing flame retardant structure, a benzoxazine structure serving as a curing agent, and a reactive group generally employed in a curing reaction.
It can be used as a curing agent for an epoxy resin and/or a resin having a phenolic hydroxyl group, whereby excellent flame retardancy and heat resistance and water resistance can be imparted to the resin(s) by addition of a small amount of the phosphorus compound, and a cured product having excellent heat and water resistance can be yielded.
The present invention has been accomplished on the basis of this finding.

Accordingly, the present invention provides the following:
(1) A phosphorus-containing benzoxazine compound represented by the formula (1):

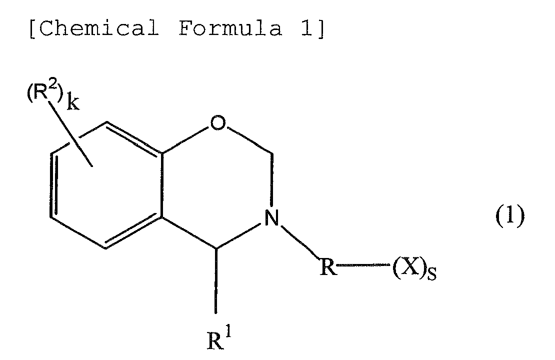

[wherein "R" represents an organic compound having a valence of (1+"s"); "X" represents a hydroxyl group, a carboxyl group, an ester group, or an unsaturated group; "s" is an integer of 1 to 5; R¹ represents a group represented by the formula (2):

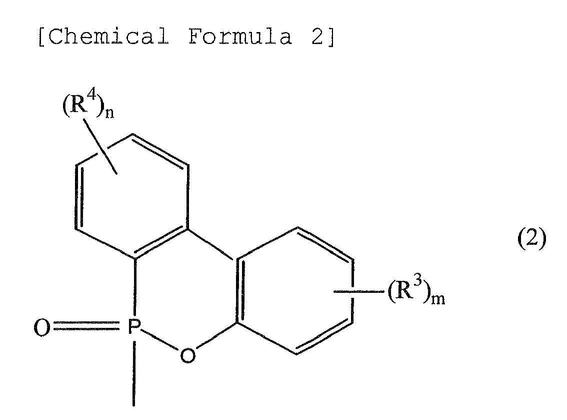

(wherein each of R³ and R⁴ independently represents a C1 to C6 alkyl group or an optionally substituted aryl group, and each of "m" and "n" is an integer of 0 to 4) or by the formula (3):

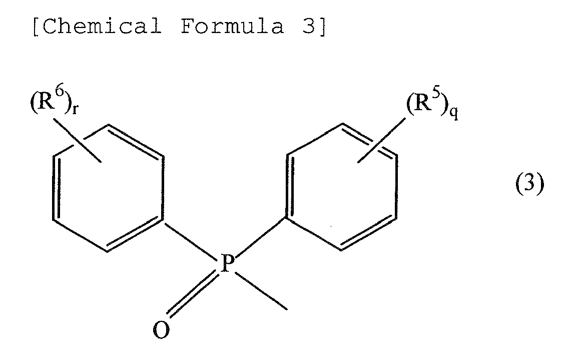

(wherein each of R⁵ and R⁶ independently represents a C1 to C6 alkyl group or an optionally substituted aryl group, and each of "q" and "r" is an integer of 0 to 5); R² represents a C1 to C6 alkyl group or an optionally substituted aryl group; and "k" is an integer of 0 to 4].
(2) A phosphorus-containing benzoxazine compound as descried in the above (1), wherein, in the formula (1), "s" is 1, and "R" is a C1 to C12 alkylene group, a C5 to C15 cycloalkylene group, or a C6 to C15 arylene group.
(3) A phosphorus-containing benzoxazine compound as described in the above (1) or (2), wherein the compound represented by the formula (1) is a compound represented by the formula (I):

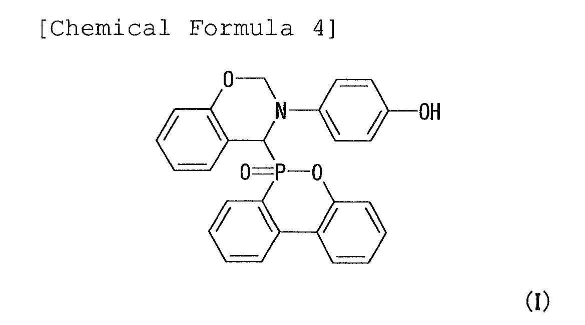

or the formula (II):

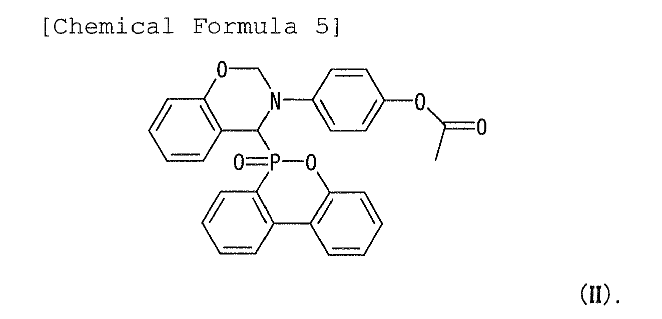

(4) A method for producing a phosphorus-containing benzoxazine compound having a structure represented by the formula (1), characterized by comprising:
reacting a 2-hydroxybenzaldehyde compound represented by the formula (4):

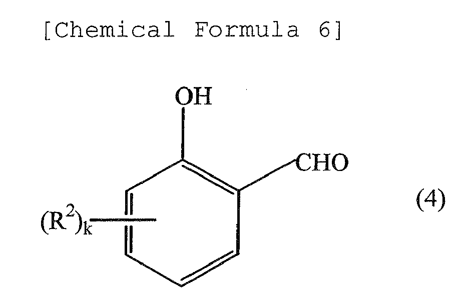

[wherein R² and "k" have the same meanings as defined in the formula (1)] with an amine compound represented by formula NH₂-R-(X)ₛ [wherein "R", "X", and "s" have the same meanings as defined in the formula (1)], to thereby yield a compound;
reacting, via addition, a phosphorus compound having a structure in which "H" is bound to "P" in a structure represented by the formula (2) or (3) with the yielded compound; and,
subsequently, reacting the addition compound with an aldehyde.
(5) A method for producing a phosphorus-containing benzoxazine compound represented by the formula (1), characterized by comprising:
reacting a 2-hydroxybenzaldehyde compound represented by the formula (4):

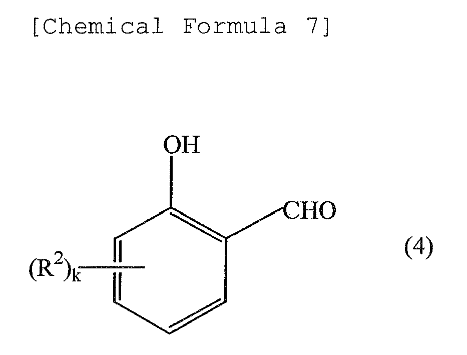

[wherein R² and "k" have the same meanings as defined in the formula (1)] with a phosphorus compound having a structure in which "H" is bound to "P" in a structure represented by the formula (2) or (3), to thereby yield a compound;
reacting, via addition, an amine compound represented by formula NH₂-R-(X)ₛ [wherein "R", "X", and "s" have the same meanings as defined in the formula (1)]; and,
subsequently, reacting the addition compound with an aldehyde.
(6) A method for producing a phosphorus-containing benzoxazine compound as described in the above (4) or (5), wherein, when "X" is a hydroxyl group, an acid anhydride is further reacted after reaction with an aldehyde.
(7) A curable resin composition having flame retardancy which contains, as essential ingredients, an epoxy resin and/or a resin having a phenolic hydroxyl group, and a phosphorus-containing benzoxazine compound as recited in any one of the (1) to (3).
(8) A cured product formed by thermally curing a curable resin composition having flame retardancy as descibed in the above (7).
(9) A laminate sheet produced by compression molding a curable resin composition having flame retardancy as descxribed in the above (8) under heating and overlaying thereon with a metal foil.
(10) A laminate sheet as described in the above (9), which is provided with a metal foil on one or both surfaces.

### Effects of the Invention

The curable resin composition of the present invention containing the phosphorus-containing benzoxazine compound and having flame retardancy exhibits high flame retardancy, even though the composition contains no halogen atom, and is excellent in heat resistance and adhesion to a copper foil. By virtue of such advantageous properties, the phosphorus-containing benzoxazine compound and the curable resin composition of the present invention containing the compound and having flame retardancy can be suitably employed for producing a laminate sheet (printed circuit boards) for electronic boards, an encapsulant for semiconductors, etc.

### Best Modes for carrying out the Invention

The present invention will be described hereinafter in detail.
The phosphorus-containing benzoxazine compound of the present invention is represented by the aforementioned formula (1).
In the formula (1), R represents a (1+"s")-valent organic compound residue, "s" is an integer of 1 to 5, preferably 1 to 3. When "s" is 1, "R" is a C1 to C12 alkylene group, a C5 to C15 cycloalkylene group, or a C6 to C15 arylene group. When "s" is ≥2, "R" is a residue formed by removing one or more hydrogen atoms from a corresponding group and having instead a bond(s).
Examples of the C1 to C12 alkylene group include methylene, ethylene, propylene, and butylenes groups, etc. Of these, ethylene and propylene groups are preferred, from the viewpoints of ease of synthesis and heat resistance of cured products obtained from the compound.
Examples of the C5 to C15 cycloalkylene group include cyclopentylene, cyclohexylene, cyclooctylene, and cyclodecylene groups, etc. Of these, cyclohexylene group is preferred, from the viewpoint of heat resistance of cured products obtained from the compound.
Examples of the C6 to C15 arylene group include phenylene, naphthylene, and anthrylene groups. Of these, phenylene group is preferred, from the viewpoints of ease of synthesis, stability of the produced compound, and heat resistance of cured products obtained from the compound.
Among the aforementioned organic compound residues, phenylene group is particularly preferred, from the viewpoints of ease of synthesis, stability of the produced compound, and heat resistance of cured products obtained from the compound.

In the formula (1), "X" represents a hydroxyl group, a carboxyl group, an ester group, or an unsaturated group. Examples of the ester group include a residue of a carboxylate ester such as an acetate. Examples of the unsaturated group include a C2 to C12 (preferably C2 to C8) alkenyl group and a styryl group. When "s" is ≥2, a plurality of "X" may be identical to or different from each other.
R² represents a C1 to C6 (preferably C1 to C2) alkyl group such as methyl or ethyl or a C6 to C15 (preferably C6 to C12) aryl group which may have a substituent. The "k" is an integer of 0 to 4, preferably 0 to 2. When "k" is an integer of 2 to 4, a plurality of R² may be identical to or different from each other.
Examples of the substituent in the aryl group include a C1 to C6 (preferably C1 to C2) alkyl group such as methyl or ethyl, a hydroxyl group, and an ester group.

R¹ is represented by the formula (2) or (3). In the formula (2), each of R³ and R⁴ independently represents a C1 to C6 alkyl group such as methyl or ethyl or an optionally substituted aryl group. The aryl group is a C6 to C15 aryl group, as described in relation to the formula (1).

When R³ and/or R⁴ in the formula (2) is an aryl group, examples of the substituent include a C1 to C6 alkyl group such as methyl or ethyl, a hydroxyl group, an ester group, and an alkoxyl group. Each of "m" and "n" is an integer of 0 to 4, preferably 0 to 2.
When "m" is an integer of 2 to 4, a plurality of R³ may be identical to or different from each other, and when "n" is an integer of 2 to 4, a plurality of R⁴ may be identical to or different from each other.

In the formula (3), each of R⁵ and R⁶ independently represents a C1 to C6 (preferably C1 to C2) alkyl group such as methyl or ethyl or an optionally substituted aryl group. The aryl group is a C6 to C15 (C6 to C12) aryl group, as described in relation to the formula (1). Examples of the substituent of the aryl group include a C1 to C6 (preferably C1 to C2) alkyl group such as methyl or ethyl, a hydroxyl group, an ester group, and an alkoxyl group. Each of "q" and "r" is an integer of 0 to 5, preferably 0 to 2.
When "q" is an integer of 2 to 5, a plurality of R⁵ may be identical to or different from each other, and when "r" is an integer of 2 to 5, a plurality of R⁶ may be identical to or different from each other.
Specific examples of the phosphorus-containing benzoxazine compound represented by the formula (1) include those represented by the formulas (I) and (II).

Hereinafter, the method for producing the phosphorus-containing benzoxazine compound of the present invention represented by the formula (1) will be described.
The phosphorus-containing benzoxazine compound can be readily synthesized from a 2-hydroxybenzaldehyde compound represented by the formula (4); an amine compound represented by NH₂-R-(X)ₛ [(hereinafter, it may be referred to simply as "amine compound") wherein "R", "X", and "s" have the same meanings as defined in the formula (1)]; a phosphorus compound having a structure in which "H" is bound to "P" in the structure represented by the formula (2) or (3) (hereinafter, it may be referred to simply as "phosphorus compound"); and an aldehyde.

In the formula (4), R² represents a C1 to C6 (preferably C1 to C2) alkyl group such as methyl or ethyl or an optionally substituted aryl group. The aryl group is a C6 to C15 (preferably C6 to C12) aryl group, as described in relation to the formula (1). Examples of the substituent of the aryl group include a C1 to C6 (preferably C1 to C2) alkyl group such as methyl or ethyl, a hydroxyl group, and an ester group.
The "k" is an integer of 0 to 4, preferably 0 to 2. When "k" is an integer of 2 to 4, a plurality of R² may be identical to or different from each other.

No particular limitation is imposed on the order of reaction steps. In a production method 1, a 2-hydroxybenzaldehyde compound is reacted with an amine compound, and a phosphorus compound is caused to be reacted (via addition reaction) to the product, followed by reacting with an aldehyde. Alternatively, in a production method 2, a 2-hydroxybenzaldehyde compound is reacted with a phosphorus compound, and an amine compound is caused to be reacted (via addition reaction) to the product, followed by reacting with an aldehyde.
It is to be noted that, when a hydroxyl-group-containing amine compound such as p-aminophenol is employed as the amine compound, such a compound represented by the formula (I) is produced.
The hydroxyl group of the formula(I) is may be reacted with an acid anhydride such as acetic anhydride so as to modify the hydroxyl group to an ester group. In this case, such a compound represented by the formula (II) is produced.
In order to stabilize the reaction to suppress side reactions, preferably, reactions in the production method 1 or 2 are generally performed in an inert solvent. Such a solvent employed has a boiling point of about 50 to about 250°C. Specific examples include alkanols such as ethanol, propanol (e.g., n-propanol, 2-propanol, and 1-methoxy-2-propanol); aromatic hydrocarbons such as toluene and xylene; alicyclic hydrocarbons such as cyclohexane; cyclic ethers such as tetrahydrofuran and 1,3-dioxorane; ethers such as dimethoxyethylene glycol; esters such as butyl acetate; and amides such as dimethylacetamide.
The amount of the solvent with respect to the produced phosphorus-containing benzoxazine compound is about 0.1 to about 5 by mass, preferably about 0.5 to about 2 by mass. When the amount of the solvent is adjusted to 0.5 or more, reaction is stabilized to suppress side reactions, whereas when the amount is adjusted to 2 or less, an increase in time and energy required for removing the solvent can be prevented.

In the production method 1, a 2-hydroxybenzaldehyde compound and an amine compound are provided such that the mole ratio of aldehyde group to amino group is adjusted to about 1/1, and the mixture is allowed to react in a solvent under reflux with dehydration. Subsequently, a phosphorus compound is added to the reaction product in such an amount that the mole ratio thereof to the formed imino groups is adjusted to about 1/1, and the mixture is allowed to react under reflux in a solvent. Thereafter, an aldehyde is added to the thus-obtained reaction product in such an amount that the mole ratio of the aldehyde to the secondary amine formed in the reaction is adjusted to about 1/1, and the mixture was is allowed to react under reflux. Finally, solvent is distilled off under reduced pressure and, if required, the product is purified through, for example, washing with water, to thereby remove unreacted substances and by-products.

In the production method 2, a 2-hydroxybenzaldehyde compound and a phosphorus compound are provided such that the mole ratio is adjusted to about 1/1, and the mixture is allowed to react in a solvent under reflux with dehydration. Subsequently, an amine compound is added to the reaction product in such an amount that the mole ratio of amino group to the formed product is adjusted to about 1/1, and the mixture is allowed to react under reflux. Thereafter, an aldehyde is added to the thus-obtained reaction product in such an amount that the mole ratio of the aldehyde to the secondary amine formed in the reaction is adjusted to the mole ratio of about 1/1, and the mixture was is allowed to react under reflux.
Finally, solvent is distilled off under reduced pressure and, if required, the product is purified through, for example, washing with water, to thereby remove unreacted substances and by-products.

Examples of the 2-hydroxybenzaldehyde compound represented by the formula (4) include 2-hydroxybenzaldehyde, 5-methyl-2-hydroxybenzaldehyde, 2,4-dihydroxybenzaldehyde, 2, 5-dihydroxybenzaldehyde, 2,3-dihydroxybenzaldehyde, and 2,3,4-trihydroxybenzaldehyde. Among them, 2-hydroxybenzaldehyde is preferably employed from the viewpoint of availability.

No particular limitation is imposed on the amine compound, so long as the compound has a primary amine moiety and a hydroxyl group, a carboxyl group, an ester group, or an unsaturated group. Specific examples include aminoalcohols such as β-aminoethyl alcohol and aminophenol; aminocarboxylic acids such as β-alanine and aminobenzoic acid; and unsaturated amines such as allylamine and aminostyrene.

No particular limitation is imposed on the phosphorus compound, so long as the compound can impart the structure represented by the formula (2) or (3). Examples of particularly preferred species thereof include 9,10-dihydro-9-oxa-10-phosphaphenanthrene-10-oxide, diphenylphosphine oxide, bis(2-methylphenyl)phosphine oxide, bis(2,5-dimethylphenyl)phosphine oxide, and bis(2,4,6-trimethylphenyl)phosphine oxide.

Specific examples of the aldehyde include formaldehyde and paraformaldehyde.

When an amino compound having a hydroxyl group such as p-aminophenol is employed, followed by reaction with an aldehyde, the hydroxyl group is converted into an ester group by use of an acid anhydride. Examples of the acid anhydride include acetic anhydride, succinic anhydride, and phthalic anhydride. The amount of the acid anhydride employed in the conversion is generally about 1/1 to 1/3 (ratio by mole) with respect to amino compound having a hydroxyl group, preferably about 1/1.1 to 1/1.5. In esterification reaction, if required, a catalyst such as p-toluenesulfonic acid, a tertiary amine compound, a quaternary ammonium salt, a phosphine compound, or a quaternary phosphonium salt may be used in an appropriate amount. The esterification reaction is generally performed at about 0 to about 120°C, preferably about 50 to about 80°C.

The thus-produced phosphorus-containing benzoxazine compound of the present invention represented by the formula (1) serves as a flame retardant and a curing agent with respect to a composition containing an epoxy resin and/or a resin having a phenolic hydroxyl group.
In other words, when the benzoxazine ring of the compound is opened through heating, the ring-opened compound is added to the resin having a phenolic hydroxyl group, whereby the resin is cured. At the same time, the formed phenolic hydroxyl group is added to an epoxy group, to thereby cure the epoxy resin.
Thus, when employed in a resin having a phenolic hydroxyl group, an epoxy group, or the like, the phosphorus-containing benzoxazine compound of the present invention is incorporated into the resin skeleton via chemical bonds. Therefore, problems such as drops in heat resistance and glass transition temperature and bleed out of a flame retardant, which would otherwise occur when an additive-type flame retardant is used, can be prevented.

Hereinafter, the flame-resistant curable resin composition, thermally cured product, and laminate sheet of the present invention will be described.
No particular limitation is imposed on the epoxy resin serving as a curable resin, and glycidyl ethers are preferably employed. Examples include bisphenol glycidyl ether, dihydroxybiphenyl glycidyl ether, dihydroxybenzene glycidyl ether, Nitrogen-containing cyclic glycidyl ether, dihydroxynaphthaleneglycidyl ether, phenol-formaldehyde polyglycidyl ether, and polyhydroxyphenol polyglycidyl ether.

Specific examples of bisphenol glycidyl ether include bisphenol A glycidyl ether, bisphenol F glycidyl ether, bisphenol AD glycidyl ether, bisphenol S glycidyl ether, and tetramethylbisphenol A glycidyl ether.

Specific examples of dihydroxybiphenyl glycidyl ether include 4,4'-biphenyl glycidyl ether, 3,3'-dimethyl-4,4'-biphenyl glycidyl ether, and 3,3',5,5'-tetramethyl-4,4'-biphenyl glycidyl ether.

Specific examples of dihydroxybenzene glycidyl ether include resorcin glycidyl ether, hydroquinone glycidyl ether, and isobutylhydroquinone glycidyl ether.

Specific examples of Nitrogen-containing cyclic glycidyl ether include triglycidyl isocyanurate and triglycidyl cyanurate.

Specific examples of dihydroxynaphthalene glycidyl ether include 1,6-dihydroxynaphthalene glycidyl ether and 2,6-dihydroxynaphthalene glycidyl ether.

Specific examples of phenol-formaldehyde polyglycidyl ether include phenol-formaldehyde polyglycidyl ether and cresol-formaldehyde polyglycidyl ether.

Specific examples of polyhydroxyphenol polyglycidyl ether include tris(4-hydroxyphenyl)methane polyglycidyl ether, tris(4-hydroxyphenyl)ethane polyglycidyl ether, tris(4-hydroxyphenyl)propane polyglycidyl ether, tris(4-hydroxyphenyl)butane polyglycidyl ether, tris(3-methyl-4-hydroxyphenyl)methane polyglycidyl ether, tris(3,5-dimethyl-4-hydroxyphenyl)methane polyglycidyl ether, tetrakis(4-hydroxyphenyl)ethane polyglycidyl ether, tetrakis(3,5-dimethyl-4-hydroxyphenyl)ethane polyglycidyl ether, and dicyclopentene-phenol-formaldehyde polyglycidyl ether.
These epoxy resins may be used singly or appropriately in combination of two or more species.

In the present invention, the phosphorus-containing benzoxazine compound and the epoxy resin are used in such a proportion that the amount epoxy group with respect to 1 equivalent of benzoxazine structure is generally adjusted to about 0.5 to about 3 equivalents, preferably about 1.0 to about 2.0 equivalents.
Through adjusting the epoxy group amount to 0.5 equivalent or higher, curing of the epoxy resin can sufficiently proceed, leading to satisfactory mechanical properties, and use of unnecessarily excessive amount of phosphorus-containing benzoxazine compound can be avoided. When the epoxy group amount is adjusted to 3 equivalents or lower, sufficient flame retardancy is ensured.

No particular limitation is imposed on the resin having a phenolic hydroxyl group serving as a curable resin, and bisphenols may be employed. Specific examples include 2,6-dihydroxynaphthalene, 2,2-bis(4-hydroxyphenyl)propane [also called bisphenol A], 2-(3-hydroxyphenyl)-2-(4'-hydroxyphenyl)propane, bis(4-hydroxyphenyl)methane [also called bisphenol F], bis(4-hydroxyphenyl)sulfone [also called bisphenol S], and phenolic resins. Specific examples of phenolic resins include phenol-formaldehyde resin, phenol-aralkyl resin, naphthol-aralkyl resin, and phenol-dicyclopentadiene copolymer resin.
These resins having a phenolic hydroxyl group may be used singly or appropriately in combination of two or more species.

When the phosphorus-containing benzoxazine compound of the present invention is employed as a curing agent for a resin having a phenolic hydroxyl group, the proportion of the amount of phenolic hydroxyl group with respect to 1 equivalent of benzoxazine structure is generally adjusted to about 0.5 to about 5 equivalents, preferably 1.0 to 3.0 equivalents.
Through adjusting the phenolic hydoxy group amount to 0.5 equivalent or higher, curing of the resin having a phenolic hydroxyl group can sufficiently proceed, leading to satisfactory mechanical properties, and use of unnecessarily excessive amount of phosphorus-containing benzoxazine compound can be avoided. When the phenolic hydoxy group amount is adjusted to 5 equivalents or lower, sufficient flame retardancy is ensured.

When the phosphorus-containing benzoxazine compound of the present invention is incorporated into a mixture of an epoxy resin and a resin having a phenolic hydroxyl group, the proportion of the amount epoxy group with respect to the total amount of phenolic hydroxyl group and benzoxazine structure is generally adjusted to about 0.5 to about 3 equivalents, preferably about 1.0 to about 2.0 equivalents.
Through adjusting the epoxy group amount to 0.5 equivalent or higher, curing of the epoxy resin and the resin having a phenolic hydroxyl group can sufficiently proceed, leading to satisfactory mechanical properties, and use of unnecessarily excessive amount of phosphorus-containing benzoxazine compound can be avoided. When the epoxy group amount is adjusted to 3 equivalents or lower, sufficient flame retardancy is ensured.

When the phosphorus-containing benzoxazine compound of the present invention is employed as a curing agent for an epoxy resin and/or a resin having a phenolic hydroxyl group, an additional curing accelerator is preferably employed in combination. The curing accelerator may be selected from those generally employed as curing accelerators for epoxy resin and/or resin having a phenolic hydroxyl group. Examples of such curing accelerators include tertiary amine compounds, quaternary ammonium salts, phosphine compounds, quaternary phosphonium salts, and imidazole compounds.
Examples of tertiary amine compounds which may be used in the invention include 1,8-diazabicyclo[5.4.0]undecene-7, dimethylbenzylamine, and tris(dimethylaminomethyl)phenol.
Examples of such quaternary ammonium salts include tetramethylammonium chloride, tetramethylammonium bromide, benzyltriethylammonium chloride, and benzyltriethylammonium bromide.
Examples of employable phosphine compounds include triphenylphosphine.
Examples of such quaternary phosphonium salts include tetrabutylphosphonium chloride and tetrabutylphosphonium bromide.
Examples of such imidazole compounds include 2-methylimidazole, 2-ethyl-4-methylimidazole, and 1-cyanoethyl-2-undecylimidazole.
These curing accelerators may be used singly or in combination of two or more species. The curing accelerator is generally added in an amount of about 0.01 to about 10 parts by mass with respect to 100 parts by mass of the resin composition, preferably 0.1 to 5 parts by mass.

In the case where a resin is imparted with flame retardancy by use of the phosphorus-containing benzoxazine compound of the present invention, the benzoxazine is employed in such an amount that phosphorus atoms are generally present in amounts of about 0.1 to about 5.0% by mass in the resin composition, preferably about 0.5 to about 2.0% by mass.

In the case where a resin is imparted with flame retardancy by use of the phosphorus-containing benzoxazine compound of the present invention, if required, an additional flame retardant may be used in combination. Examples of the flame retardant include metal hydroxides such as aluminum hydroxide and phosphorus-containing compounds such as phosphate esters and phosphazene. There may also be employed, as disclosed in the aforementioned JP 11-279258 A, an epoxy resin containing 20% by mass or more a novolak epoxy resin and a compound produced through reaction between a quinone compound and a compound having a structure in which "H" is bound to "P" in the structure represented by formulas (2) and/or (3).

The flame-resistant curable resin composition containing the phosphorus-containing benzoxazine compound of the present invention may further contain, in accordance with needs, additives such as a filler, a coupling agent, a lubricant, a mold-releasing agent, a plasticizer, a colorant, and a thickener.

By curing the thus-prepared curable resin composition containing the phosphorus-containing benzoxazine compound of the present invention and having flame retardancy under the following conditions, a thermally cured product is obtained.
The curing temperature and time are generally about 160 to about 240°C and about 30 to about 180 minutes, preferably about 180 to about 220°C and about 60 to about 120 minutes. By controlling the curing temperature to 160°C or higher and the curing time to 30 minutes or longer, curing sufficiently proceeds. By controlling the temperature and time to 240°C or lower and 180 minutes or shorter, discoloring and thermal deterioration (in physical properties) of cured products are prevented, and a drop in productivity is prevented.

When a thermally cured product is produced through thermal reaction of the curable resin composition containing the phosphorus-containing benzoxazine compound of the present invention and having flame retardancy, known molding techniques may be employed. Examples of such molding techniques include melt-cast molding, compression molding (heat pressing by means of a compression molding machine), transfer molding (injecting a plasticized molding material into a cavity of a metal mold), laminated molding (stacking several prepreg sheets and heat-compressing the laminate for curing to produce a laminated cured product), matched die molding (impregnating a preform with resin, followed by compression molding), SMC method, BMC method, pultrusion molding (unidirectionally impregnating a fiber filament with resin, followed by curing in a die), filament winding (winding resin-impregnated roving by a core), and RIM method.

The curable resin composition containing the phosphorus-containing benzoxazine compound of the present invention and having flame retardancy is more excellent in flame retardancy and heat resistance, as compared with resin compositions employing a conventional flame retardant or based on a conventional flame retardant technique and cured products obtained from such resin compositions. When metal foil is laminated on the curable resin composition of the present invention, excellent adhesion therebetween can be attained.
By virtue of these excellent characteristics, the phosphorus-containing benzoxazine compound of the present invention and the curable resin composition employing the compound and having flame retardancy can be suitably employed as laminate sheets for electronic boards (printed circuit boards), semiconductor encapsulating material, electronic material for printed circuit boards, etc.
The present invention also provides a laminate sheet, which is formed through compression molding with heating the aforementioned curable resin composition of the present invention having flame retardancy. The laminate sheet may be provided, on one or both surfaces thereof, with a metal foil. The laminate sheet is suitably employed as substrates for printed circuit boards, etc.
No particular limitation is imposed on the metal forming the metal foil so long as the metal is of general uses. Examples of the metal include aluminum, copper, nickel, and alloys thereof. Among them, a copper foil and a copper-base alloy foil are preferred, from the viewpoints of physical and electric performance, etc.

The curable resin composition containing the phosphorus-containing benzoxazine compound of the present invention may also be employed as a material with which a fiber reinforce substrate (e.g., carbon fiber, glass fiber, aramide fiber, polyester fiber, nylon fiber, or SiC fiber) is impregnated. The amount of fiber reinforce substrate may be appropriately predetermined. For example, the amount is preferably 5 to 500 parts by mass with respect to 100 parts by mass of the resin composition, more preferably 10 to 300 parts by mass. Moreover, the curable resin composition containing the phosphorus-containing benzoxazine compound of the present invention and having flame retardancy can be applied not only to electronic material but also to automobile parts, OA (office automation)-related parts, etc.

### Examples

The present invention will be described hereinafter in detail by way of Examples, Comparative Examples, and Application Examples, which should not be construed as limiting the invention thereto.

### Example 1

An amine compound, p-aminophenol, (109 g, 1.0 mol) and a 2-hydroxybenzaldehyde compound, 2-hydroxybenzaldehyde, (122 g, 1.0 mol) were added to 2-propanol (b.p.: 82.4°C) (477 g) as a solvent, and the mixture was allowed to react for three hours under reflux with dehydration. Subsequently, a phosphorus compound having a P-H group, 9,10-dihydro-9-oxa-10-phosphaphenanthrene-10-oxide, (216 g, 1.0 mol) was added thereto, and the resultant mixture was allowed to react for three hours under reflux. Subsequently, an aldehyde, paraformaldehyde, (30 g, 1.0 mol) was added thereto, followed by reaction for six hours under reflux. Finally, the solvent (2-propanol) was distilled off under reduced pressure, whereby a phosphorus-containing benzoxazine compound (A-1) was yielded as a brown solid. Through an elemental analysis, a mass spectrometry, ¹H-NMR, and an infrared absorption spectrometry, compound A-1 was identified as a compound represented by the formula (I).
The phosphorus element content was found to be 7.1% (theoretical value: 7.0%) through the elemental analysis.
The molecular weight was found to be 441.0 (calculated: 441.42) through the mass spectrometry (M/Z), and ¹H-NMR (DMSO-d₆) absorption peaks were assigned as follows: 5.5 ppm (1H), 5.8 ppm (2H), 7.0 ppm (4H), 7.3 ppm (2H), 7.6 to 8.1 ppm (6H), 8.3 to 8.4 ppm (2H), 8.8 ppm (2H), and 9.6 ppm (1H).
The absorption peaks (cm⁻¹) observed in the infrared absorption spectrometry were as follows: 3264, 3061, 3025, 1676, 1606, 1594, 1582, 1514, 1488, 1476, 1449, 1430, 1394, 1374, 1312, 1261, 1227, 1199, 1177, 1148, 1115, 1082, 1048, 1038, 978, 926, 863, 836, 806, 789, 752, 718, 708, and 684.

### Example 2

p-Aminophenol (109 g, 1.0 mol) and 2-hydroxybenzaldehyde (122 g, 1.0 mol) were added to 2-propanol (477 g), and the mixture was allowed to react for three hours under reflux with dehydration. Subsequently, 9,10-dihydro-9-oxa-10-phosphaphenanthrene-10-oxide (216 g, 1.0 mol) was added thereto, and the resultant mixture was allowed to react for three hours under reflux. Subsequently, paraformaldehyde (30 g, 1.0 mol) was added thereto, followed by reaction for six hours under reflux. Then, through heating and reducing pressure, the solvent was substituted by toluene, and an acid anhydride, acetic anhydride, (128 g, 1.25 mol) was caused to react, whereby a phenolic hydroxyl group was acetylated. Finally, the acetylated product was washed with ion-exchange water and dried, whereby a phosphorus-containing benzoxazine compound (A-2) was yielded. Compound A-2 was identified as a compound represented by the formula (II). The phosphorus content was found to be 6.3% by mass.

### Example 3

9,10-Dihydro-9-oxa-10-phosphaphenanthrene-10-oxide (216 g, 1.0 mol) and 2-hydroxybenzaldehyde (122 g, 1.0 mol) were added to 1-methoxy-2-propanol (477 g), and the mixture was allowed to react at 80°C for three hours with dehydration. Subsequently, an amine compound, p-aminophenol, (109 g, 1.0 mol) was added thereto, and the resultant mixture was allowed to react for three hours.
Subsequently, paraformaldehyde (30 g, 1.0 mol) was added thereto, followed by reaction at 80°C for six hours under reflux. Finally, solvent was distilled off under reduced pressure, whereby a phosphorus-containing benzoxazine compound (A-3) was yielded as a brown solid. Through the ¹H-NMR and infrared absorption spectrometry, compound A-3 was identified as a compound represented by the formula (I).
¹H-NMR (DMSO-d₆) absorption peaks were assigned as follows: 5.5 ppm (1H), 5.8 ppm (2H), 7.0 ppm (4H), 7.4 ppm (2H), 7.6 to 8.1 ppm (6H), 8.3 to 8.4 ppm (2H), 8.8 ppm (2H), and 9.6 ppm (1H).
The absorption peaks (cm⁻¹) observed in the infrared absorption spectrometry were as follows: 3264, 3061, 3025, 1676, 1606, 1594, 1582, 1514, 1488, 1476, 1449, 1430, 1394, 1374, 1312, 1261, 1227, 1199, 1177, 1148, 1115, 1082, 1048, 1038, 978, 926, 863, 836, 806, 789, 752, 718, 708, and 684.

### Comparative Example 1

An amine compound, aniline, (93 g, 1.0 mol) and 2-hydroxybenzaldehyde (122 g, 1.0 mol) were added to 2-propanol (459 g), and the mixture was allowed to react for three hours under reflux with dehydration. Subsequently, 9,10-dihydro-9-oxa-10-phosphaphenanthrene-10-oxide (216 g, 1.0 mol) was added thereto, and the resultant mixture was allowed to react for three hours under reflux.
Subsequently, paraformaldehyde (30 g, 1.0 mol) was added thereto, followed by reaction for six hours under reflux. Finally, 2-propanol was distilled off under reduced pressure, whereby a phosphorus-containing benzoxazine compound (A-4) was yielded as a brown solid. Through ¹H-NMR and infrared absorption spectrometry, compound A-4 was identified as a compound represented by the formula (III) shown hereinafter.
¹H-NMR (CDCl₃) absorption peaks were assigned as follows: 5.2 ppm (1H), 5.5 ppm (2H), 6.7 ppm (2H), 6.8 ppm (2H), 7.0 to 7.4 ppm (6H), 7.5 ppm (2H), 7.8 ppm (2H), and 8.3 ppm (2H).
The absorption peaks (cm⁻¹) observed in the infrared absorption spectrometry were as follows: 3061, 2905, 1594, 1581, 1489, 1472, 1446, 1428, 1365, 1314, 1268, 1257, 1224, 1212, 1202, 1187, 1160, 1148, 1117, 1082, 1042, 1029, 1000, 977, 955, 909, 878, 808, 781, 774, 761, 751, 717, 710, 696, and 687.

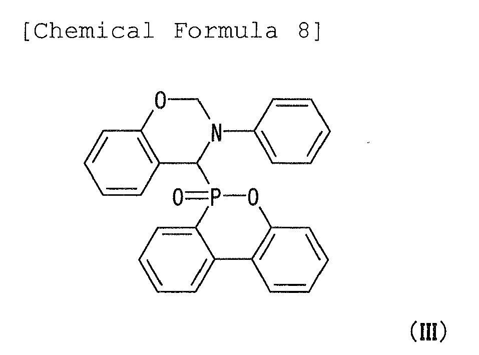

### Comparative Example 2

An amine compound, benzylamine, (107 g, 1.0 mol) and 2-hydroxybenzaldehyde (122 g, 1.0 mol) were added to 2-propanol (477 g), and the mixture was allowed to react for three hours under reflux with dehydration. Subsequently, 9,10-dihydro-9-oxa-10-phosphaphenanthrene-10-oxide (216 g, 1.0 mol) was added thereto, and the resultant mixture was allowed to react for three hours under reflux. Subsequently, paraformaldehyde (30 g, 1.0 mol) was added thereto, followed by reaction for six hours under reflux. Finally, 2-propanol was distilled off under reduced pressure, whereby a phosphorus-containing benzoxazine compound (A-5) was yielded as a brown solid. The compound A-5 was identified as a compound represented by the formula (IV) shown hereinafter. The compound was found to have a phosphorus content of 7.0% by mass.

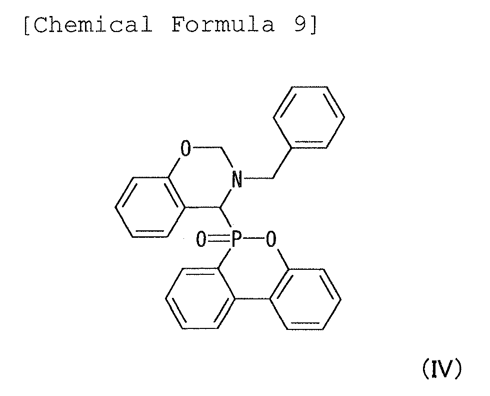

### ≪Application Examples 1 and 2 and Comparative Application Examples 1 to 5

### <Formulations of curable resin compositions having flame retardancy>

Ingredients shown in Table 1 were dissolved in a solvent at proportions shown in Table 1 through the below-described procedure, to thereby prepare varnishes. Each varnish was cured under the below-described conditions, to thereby prepare double-side-copper-clad laminate sheet pieces. Peel strength, flame retardancy (UL), Tg (DMA method), heat resistance, and solder resistance of the test pieces were measured. Evaluation results are shown in Table 1.
It is to be noted that the units "part(s)" and "%" are based on "mass" in Table 1.

[Table 1]

**Table 1-1**

| | Appln. Ex. 1 | Appln. Ex. 2 |
|---|---|---|
| Epoxy resin (N-680) | 60 | 58 |
| Epoxy resin (ZX-1548-4) | | |
| Phenolic compound (BP-F) | 10 | 9 |
| Phenolic compound (LA-7051) | | |
| Phosphorus-containing benzoxazine compound (A-1) | 30 | |
| The same as above (A-2) | | 33 |
| Comparative Phosphorus-containing benzoxazine compound (A-4) | | |
| The same as above (A-5) | | |
| Comparative Phosphorus-containing compound (PX-200) | | |
| Methoxypropanol | 50 | 33 |
| MEK | | 33 |
| Curing accelerator (C11Z-CN) | 1 | 1 |
| Phosphorus content in curable resin composition (%) | 2.1 | 2.1 |
| Flame retardancy (UL-94) | V-0 | V-0 |
| Peel strength (kN/m) | 1.5 | 1.4 |
| Tg (DMA, °C) | 182 | 180 |
| Heat resistance (°C) | 250 | 250 |
| Temperature of 2%-weight loss (°C) | 349 | 346 |
| Solder resistance | fair | fair |

**Table 1-2**

| | Comp. Appln. Ex. 1 | Comp. Appln. Ex. 4 | Comp. Appln. Ex. 5 | Comp. Appln. Ex. 6 | Comp. Appln. Ex. 7 |
|---|---|---|---|---|---|
| Epoxy resin (N-680) | 55 | 55 | | | 53 |
| Epoxy resin (ZX-1548-4) | | | 50 | 50 | |
| Phenolic compound (BP-F) | 15 | 15 | 34 | 20 | 25 |
| Phenolic compound (LA-7051) | | | | 30 | |
| Phosphorus-containing benzoxazine compound (A-1) | | | | | |
| The same as above (A-2) | | | | | |
| Comparative Phosphorus-containing benzoxazine compound (A-4) | 30 | | | | |
| The same as above (A-5) | | 30 | | | |
| Comparative Phosphorus-containing compound (PX-200) | | | | | 22 |
| Methoxypropanol | 25 | 25 | 25 | 33 | 33 |
| MEK | 25 | 25 | 25 | 33 | 33 |
| Curing accelerator (C11Z-CN) | 1 | 1 | 1 | 1 | 1 |
| Phosphorus content in curable resin composition (%) | 2.1 | 2.1 | 2.0 | 2.0 | 2.0 |
| Flame retardancy (UL-94) | V-0 | V-0 | V-0 | V-0 | V-0 |
| Peel strength (kN/m) | 1.6 | 1.5 | 1.4 | 1.3 | 1.5 |
| Tg (DMA, °C) | 149 | 136 | 136 | 152 | 79 |
| Heat resistance (°C) | 240 | 230 | 250 | 240 | 230 |
| Temperature of 2%-weight loss (°C) | 340 | 336 | 376 | 355 | 341 |
| Solder resistance | fair | fair | fair | fair | fair |

The following compounds were employed as the epoxy resins and the curing agents in Table 1.

### <Epoxy resins>

(1) Cresol novolak epoxy resin (EPICLON N-680, a product of Dainippon Ink and Chemicals, Inc., epoxy equivalent: 208 g/equivalent)
(2) Phosphorus compound-modified novolak epoxy resin (ZX-1548-4, a product of Tohto Kasei Co., Ltd., epoxy equivalent= 407 g/equivalent)

### <Resins having a phenolic hydroxyl group>

(1) Bisphenol F (BP-F, a product of Honshu Chemical Industry Co., Ltd., OH equivalent= 100 g/equivalent)
(2) Aminotriazine novolak resin (Phenolite LA-7051, a product of Dainippon Ink and Chemicals, Inc., OH equivalent: 124 g/equivalent)

### <Phosphorus-containing compounds>

(1) Phosphorus-containing benzoxazine compounds (A-1) to (A-4)
(2) 1,3-Phenylenebis(di-2,6-xylenylphosphate) (a phosphate ester-based flame retardant, a product of Daihachi Chemical Industry Co, Ltd., PX-200, Phosphorus content: 9.0% by mass)

### <Curing accelerator>

1-Cyanoethyl-2-undecylimidazole (a curing accelerator, a product of Shikoku Chemicals Co., Cresol C11Z-CN)

### [Preparation of varnishes]

Each of the varnishes was prepared by dissolving the ingredients at proportions shown in Table 1 in a solvent; i.e., methoxypropanol or a methoxypropanol-methyl ethyl ketone equiamount mixture; adding a curing accelerator (C11Z-CN) to the solution; and adjusting the non-volatile (N.V.) content of the final curable resin composition to 60% by mass or 66% by mass.
The amount of curing accelerator was adjusted to 1 part by mass with respect to 100 parts by mass of resins (an epoxy resin and a curing agent).

### [Conditions under which Laminate Sheets were prepared]

A glass cloth piece (Glass cloth "WE18K105,"a product of Nitto Boseki Co., Ltd.) (thickness: about 180 µm) was impregnated with each of the varnishes prepared in Application Examples 1 to 2 and Comparative Application Examples 1 to 5, and solvents was distilled off to dryness.
Then, the piece was preliminarily dried at 120°C for 3 minutes, then at 160°C for 3 minutes, to thereby prepare a prepreg. Copper foil (thickness: about 18 µm, JTC1/20Z, a product of Nikko Material) was laminated on each surface of the prepreg, followed by compression molding at 3.92 MPa and 200°C for 60 minutes, to thereby prepare a laminate sheet. The thus-produced laminate sheet was found to have a thickness of about 0.2 mm and a resin content of about 40% by mass.

### [Evaluated Physical Properties and Test Conditions]

(1) Flame retardancy
   It was measured by in accordance with UL-94 Vertical Burning Test.
(2) Glass transition temperature (Tg)
   It was measured through the DMA method (temperature elevation rate: 3°C/min) by means of RTM-1T (a product of ORIENTEC, Co.).
(3) Weight loss initiating temperature
   It was measured by means of TG/DTA6200 (a product of SII, Co.) under a flow of nitrogen at a temperature elevation rate of 10°C/min.
(4) Elemental analysis
   Phosphorus content of a sample was measured by decomposing the sample with sulfuric acid and nitric acid, followed by ICP spectrometry.
(5) Mass spectrometry
   It was measured by means of Thermofinnigan LCQ Advantage.
(6) ¹H-Nuclear magnetic resonance spectrometry (¹H-NMR)
   It was measured by means of JNM-LA300 (a product of JEOL) by use of tetramethylsilane as an internal standard.
(7) Infrared spectrometry
   It was measured by means of a Fourier transformation infrared spectrometer (Spectrum One, a product of Perkin Elmer).
(8) Peel strength
   It was measured by in accordance with JIS-C6481.
(9) Heat resistance
   It was measured by in accordance with JIS-C6481, with a testing time was 60 minutes. The temperature at which all samples (n=3) passed the test was recorded.
(10) Solder resistance
   It was measured by in accordance with JIS-C6481. When a laminate sheet sample was immersed in a solder at 260°C for 120 seconds, occurrence of swell was visually observed. The sample exhibiting no swell was evaluated as "fair".

As is clear from Table 1, laminate sheets produced through heat curing of the curable resin composition containing the phosphorus-containing benzoxazine compound of the present invention and having flame retardancy exhibit excellent flame retardancy and are excellent in heat resistance and adhesion to a copper foil.

### Industrial Applicability

The curable resin composition containing the phosphorus-containing benzoxazine compound of the present invention and having flame retardancy is suitably used in the electronic material field, and particularly suitable for an encapsulant for semiconductors, a laminate sheet, a coating material, a composite material, etc.

## Claims

1. A phosphorus-containing benzoxazine compound represented by the formula (1): 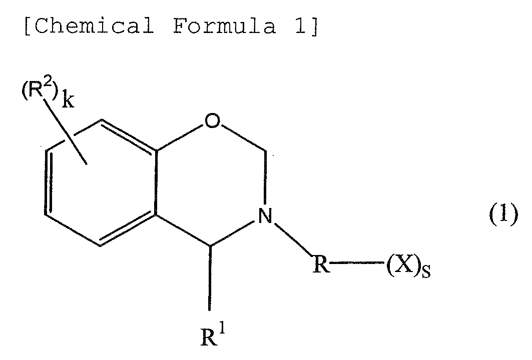 [wherein "R" represents an organic compound having a valence of (1+"s"); "X" represents a hydroxyl group, a carboxyl group, an ester group, or an unsaturated group; "s" is an integer of 1 to 5; R¹ represents a group represented by the formula (2): 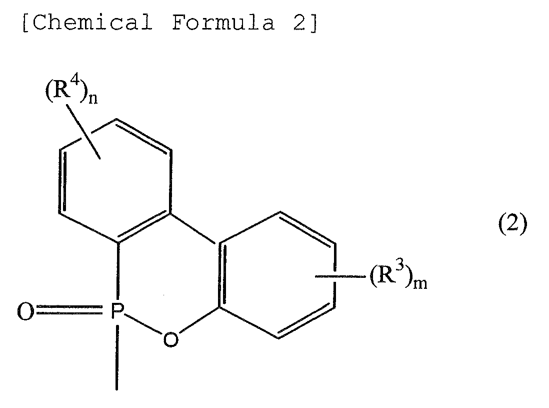 (wherein each of R³ and R⁴ independently represents a C1 to C6 alkyl group or an optionally substituted aryl group, and each of "m" and "n" is an integer of 0 to 4) or by the formula (3): 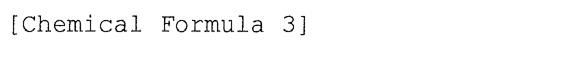 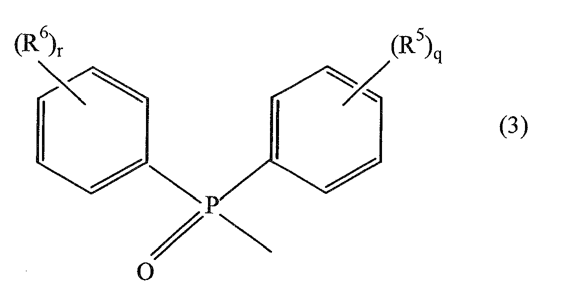 (wherein each of R⁵ and R⁶ independently represents a C1 to C6 alkyl group or an optionally substituted aryl group, and each of "q" and "r" is an integer of 0 to 5); R² represents a C1 to C6 alkyl group or an optionally substituted aryl group; and "k" is an integer of 0 to 4].

2. A phosphorus-containing benzoxazine compound as claimed in claim 1, wherein, in the formula (1), "s" is 1, and "R" is a C1 to C12 alkylene group, a C5 to C15 cycloalkylene group, or a C6 to C15 arylene group.

3. A phosphorus-containing benzoxazine compound as claimed in claim 1 or 2, wherein the compound represented by the formula (1) is a compound represented by the formula (I): 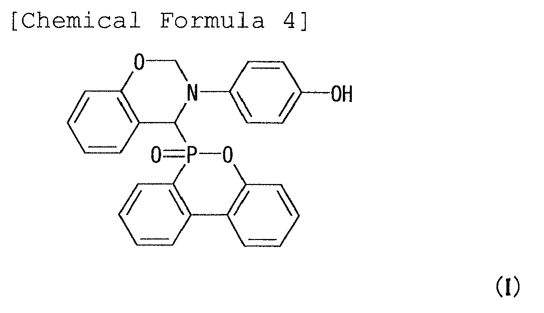 or the formula (II):

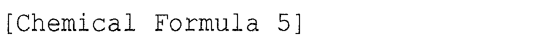

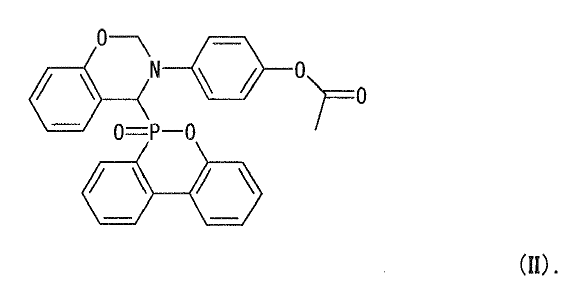

4. A method for producing a phosphorus-containing benzoxazine compound having a structure represented by the formula (1), **characterized by** comprising:
reacting a 2-hydroxybenzaldehyde compound represented by the formula (4): 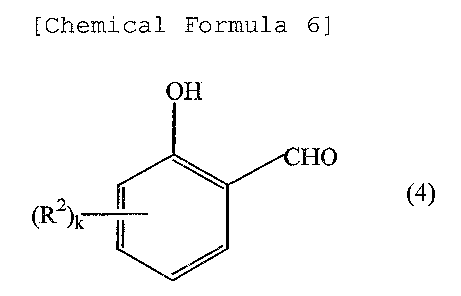 [wherein R² and "k" have the same meanings as defined in the formula (1)] with an amine compound represented by formula NH₂-R-(X)ₛ [wherein "R", "X", and "s" have the same meanings as defined in the formula (1)], to thereby yield a compound;
reacting, via addition, a phosphorus compound having a structure in which "H" is bound to "P" in a structure represented by the formula (2) or (3) with the yielded compound; and,
subsequently, reacting the addition compound with an aldehyde.

5. A method for producing a phosphorus-containing benzoxazine compound represented by the formula (1), **characterized by** comprising:
reacting a 2-hydroxybenzaldehyde compound represented by the formula (4): 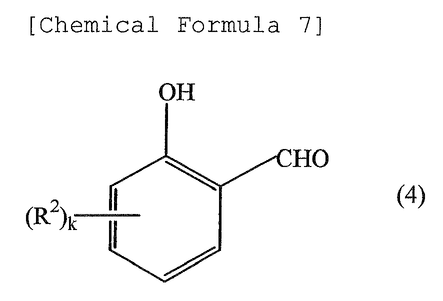 [wherein R² and "k" have the same meanings as claimed in the formula (1)] with a phosphorus compound having a structure in which "H" is bound to "P" in a structure represented by the formula (2) or (3), to thereby yield a compound;
reacting, via addition, an amine compound represented by formula NH₂-R-(X)ₛ [wherein "R", "X", and "s" have the same meanings as defined in the formula (1)]; and,
subsequently, reacting the addition compound with an aldehyde.

6. A method for producing a phosphorus-containing benzoxazine compound as claimed in claim 4 or 5, wherein, when "X" is a hydroxyl group, an acid anhydride is further reacted after reaction with an aldehyde.

7. A curable resin composition having flame retardancy which contains, as essential ingredients, an epoxy resin and/or a resin having a phenolic hydroxyl group, and a phosphorus-containing benzoxazine compound as claimed in any one of claims 1 to 3.

8. A cured product formed by thermally curing a curable resin composition having flame retardancy as claimed in claim 7.

9. A laminate sheet produced by compression molding a curable resin composition having flame retardancy as claimed in claim 8 under heating and overlaying thereon with a metal foil.

10. A laminate sheet as claimed in claim 9, which is provided with a metal foil on one or both surfaces.
